# FASCICULE DE BREVET EUROPEEN

(11) **EP 3 362 763 B1**
(45) Date de publication et mention de la délivrance du brevet: **14.08.2019**
(21) Numéro de dépôt: 16794684.7
(22) Date de dépôt: 07.10.2016
(51) Int. Cl.: G01B 11/08, A61B 5/107, A61M 1/06

(54) **METHODE DE DETERMINATION D'UNE TAILLE DE TETERELLE, ET PRODUIT PROGRAMME INFORMATIQUE**
VERFAHREN ZUR BESTIMMUNG EINER BRUSTPUMPENGRÖSSE UND COMPUTERPROGRAMMPRODUKT
METHOD FOR DETERMINING A BREAST PUMP SIZE, AND COMPUTER PROGRAM PRODUCT

(30) Priorité: 12.10.2015 FR 1559687
(43) Date de publication de la demande: 22.08.2018
(73) Titulaire: LA DIFFUSION TECHNIQUE FRANCAISE, 42000 Saint Etienne (FR)
(72) Inventeur: CHANTREL, Gilles, 42100 Saint-Etienne (FR); MASSARDIER, Jean-Philippe, 42100 Saint-Etienne (FR)
(74) Mandataire: Cabinet Laurent & Charras
(86) Numéro de dépôt international: PCT/FR2016/052589
(87) Numéro de publication internationale: WO 2017/064399

(56) Documents cités:
- US-A1- 2011 295 112
- US-A1- 2014 300 722
- US-B1- 8 776 387
- Anonymous: "Choisir la bonne taille de téterelle PersonalFit", , 6 mai 2015 (2015-05-06), page 1, XP055279076, Extrait de l'Internet: URL:http://www.medela.com.medela5.nine.ch/ CA/dms/ca/breastfeeding/images/breastcare/ untitled/2908489A_PersonalFit_FR_Breastshi eld-size-infosheet_sm/2908489A_PersonalFit _FR_Breastshield size infosheet_sm.pdf [extrait le 2016-06-09]

## Description

La présente invention concerne une méthode de détermination d'une taille de téterelle, par une utilisatrice munie d'un terminal mobile personnel, tel qu'un téléphone ou une tablette numérique (« smartphone » ou « tablet » en anglais).

L'invention concerne également un produit programme informatique pour la mise en oeuvre d'une telle méthode.

Le domaine de l'invention est celui des équipements pour l'allaitement des nourrissons, en particulier les téterelles pour l'expression du lait.

Une téterelle est un embout utilisé pour le recueil du lait à l'aide d'un tire-lait, qui est ensuite donné au nourrisson, par exemple à l'aide d'un biberon. Comme il existe différentes dimensions et formes de sein, il existe différentes dimensions et formes de téterelles. Afin que la téterelle présente un fonctionnement optimal, il est important de déterminer la taille adaptée à la maman.

Si la téterelle présente une taille appropriée et est bien ajustée sur le mamelon, les canaux galactophores du sein ne sont pas comprimés outre mesure durant l'expression du lait. Ainsi, le sein se vide de façon optimale et la production de lait est maintenue. En outre, la présence de la téterelle permet de réduire une éventuelle douleur au sein.

En revanche, si la téterelle présente une taille inappropriée et est mal ajustée sur le mamelon, les canaux galactophores du sein peuvent se bloquer. Le sein n'est pas vidé en entier et le réflexe d'éjection du lait peut être altéré, voire inhibé. En outre, les frottements peuvent causer une douleur et même des lésions au niveau du mamelon.

Pour déterminer la taille optimale de téterelle convenant à une maman, il est connu de mesurer la taille du mamelon et de la comparer avec les tailles de téterelles disponibles sur le marché. Par exemple, la mesure est réalisée avec une réglette en papier ou en plastique, puis la sélection est réalisée dans une brochure ou un catalogue.

Le but de la présente invention est de proposer une méthode améliorée de détermination d'une taille de téterelle.

A cet effet, l'invention a pour objet selon la revendication 1 une méthode de détermination d'une taille de téterelle, par une utilisatrice munie d'un terminal mobile personnel équipé d'un écran et d'un dispositif de capture d'image, la méthode comprenant les étapes successives suivantes :
- une étape consistant à caractériser un repère de dimensions déterminées, affiché sous forme d'un repère numérique sur l'écran du terminal ;
- une étape consistant à comparer les dimensions d'une représentation de mamelon de l'utilisatrice avec des cercles de différents diamètres affichés à côté du repère numérique sur l'écran du terminal, alors que le repère est positionné à côté d'un mamelon de l'utilisatrice ; et
- une étape consistant à sélectionner la taille de téterelle convenant à l'utilisatrice, en fonction du cercle retenu à la fin de l'étape précédente.

Ainsi, l'invention permet à l'utilisatrice de déterminer la taille de téterelle qui lui convient le mieux, de manière simple, pratique et ludique. Avantageusement, la méthode selon l'invention peut être mise en oeuvre à l'aide d'une application dédiée, exécutée sur le terminal de l'utilisatrice. En comparaison avec une réglette, qui présente des informations figées dans le temps, l'application peut être mise à jour en fonction de l'évolution des téterelles disponibles sur le marché.

D'autres caractéristiques avantageuses de l'invention, prises isolément ou en combinaison, sont détaillées ci-après :
- L'étape consistant à caractériser le repère de dimensions déterminées comprend les sous-étapes successives suivantes : une sous-étape consistant à positionner le repère face au dispositif de capture d'image, de sorte que le repère est automatiquement affiché sous forme de repère numérique sur l'écran ; une sous-étape consistant à positionner le repère numérique dans un cadre affiché sur l'écran ; et une sous-étape consistant à actionner le dispositif de capture d'image, lorsque le repère numérique est positionné dans le cadre.
- L'étape consistant à comparer les dimensions de la représentation de mamelon avec les cercles de différents diamètres comprend les sous-étapes successives suivantes : une sous-étape consistant à générer les cercles de différents diamètres à côté du repère numérique sur l'écran ; une sous-étape consistant à positionner le repère à côté d'un mamelon de l'utilisatrice, de sorte que le repère numérique et les cercles sont positionnés à côté de la représentation de mamelon sur l'écran ; et une sous-étape consistant à déplacer le repère autour du mamelon de l'utilisatrice, jusqu'à identifier le cercle dont le diamètre s'accorde avec les dimensions de la représentation de mamelon sur l'écran.

- Optionnellement, l'étape consistant à comparer les dimensions de la représentation de mamelon avec les cercles de différents diamètres comprend une sous-étape consistant à afficher une page d'aide, au choix de l'utilisatrice.
- L'étape consistant à sélectionner la taille de téterelle convenant à l'utilisatrice comprend les sous-étapes successives suivantes : une sous-étape consistant à sélectionner un cercle coloré en appuyant sur l'écran, en fonction du cercle retenu par l'utilisatrice ; et une sous-étape consistant à indiquer à l'utilisatrice la taille de téterelle qui lui convient le mieux.
- Dans un mode de réalisation particulier, l'étape consistant à sélectionner la taille de téterelle convenant à l'utilisatrice comprend : une sous-étape additionnelle consistant à sélectionner un gabarit de téterelle en appuyant sur l'écran, en fonction de la forme du sein de l'utilisatrice ; et la sous-étape consistant à indiquer à l'utilisatrice la taille et également le gabarit de téterelle qui lui conviennent le mieux.
- Dans la sous-étape consistant à indiquer à l'utilisatrice la taille de téterelle qui lui convient le mieux, un récapitulatif mentionnant la taille, le gabarit (le cas échéant) et une référence de téterelle est affiché sur l'écran.
- Le repère présente les dimensions standards d'une carte à puce ID-1.

L'invention a également pour objet selon la revendication 8 un produit programme informatique, comprenant des portions de code de programme enregistrées sur le terminal de l'utilisatrice, pour la mise en oeuvre des étapes de la méthode de détermination mentionnée ci-dessus, lorsque ledit programme est exécuté sur le terminal de l'utilisatrice.

L'invention sera mieux comprise à la lecture de la description qui va suivre, donnée uniquement à titre d'exemple non limitatif et faite en référence aux dessins annexés sur lesquels :
- la figure 1 est une vue en perspective d'une téterelle ;
- la figure 2 est une vue en perspective, à plus petite échelle, de téterelles de différentes tailles couplées à des dispositifs de recueil de lait ;
- la figure 3 montre des disques de différentes tailles, correspondant aux téterelles de différentes tailles ;
- la figure 4 est un ordinogramme illustrant la méthode de détermination conforme à l'invention ; et
- les figures 5 à 12 montrent différentes étapes de la méthode de détermination conforme à l'invention.

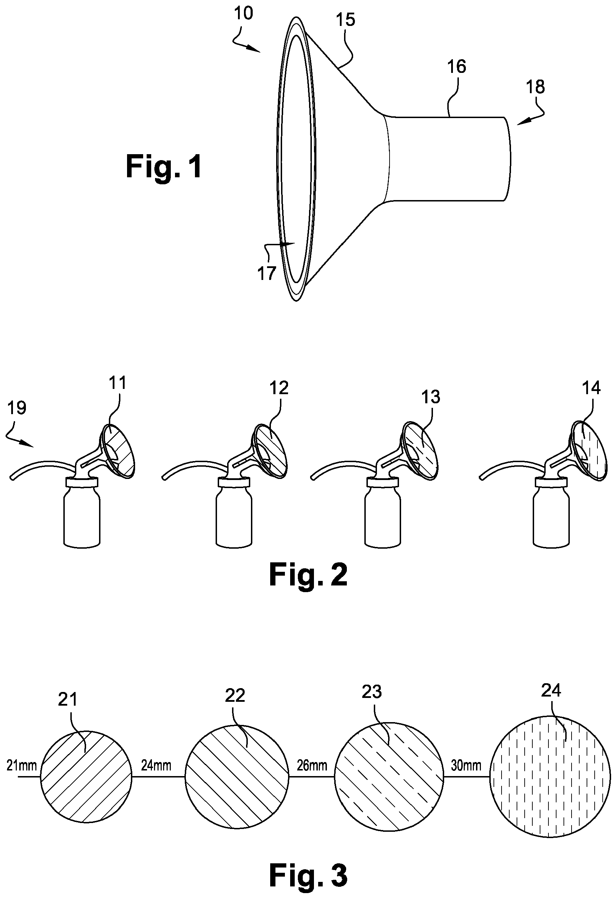

Sur la figure 1 est représentée une téterelle 10, comprenant une partie 15 de forme tronconique et une partie 16 de forme cylindrique reliées entre elles. La partie 15 comporte une ouverture large 17 et est prévue pour être positionnée sur le sein, autour de l'aréole. La partie 16 comporte une ouverture étroite 18 pour la sortie du lait et est prévue pour être positionnée autour du mamelon, sans contact pour éviter les frottements.

La téterelle 10 peut présenter toute autre configuration adaptée à l'application visée. Par exemple, la partie 15 peut être légèrement bombée, tandis que la partie 16 peut être tronconique au lieu de cylindrique.

Sur la figure 2 sont représentées différentes téterelles 11, 12, 13 et 14 de tailles croissantes, chacune associée à un dispositif 19 de recueil du lait. Chaque dispositif 19 comprend un biberon, un capuchon monté sur le biberon et connecté à la téterelle, et un conduit qui s'étend depuis le capuchon jusqu'à une pompe à lait non représentée.

Les téterelles 11, 12, 13 et 14 ont différentes tailles, c'est-à-dire que leurs parties 15 et 16 ont différentes tailles, pour s'adapter à différentes tailles de sein. Les téterelles 11, 12, 13 et 14 peuvent être proposées avec différentes couleurs pour faciliter leur différenciation, par exemple violet, orange, vert et bleu, respectivement.

Sur la figure 3 sont représentés différents disques 21, 22, 23 et 24 de tailles croissantes, dont les diamètres et les couleurs correspondent à ceux des téterelles 11, 12, 13 et 14, respectivement. A titre d'exemple préféré mais non limitatif, les disques colorés 21, 22, 23 et 24 peuvent avoir des diamètres de 21, 24, 26 et 30 millimètres, respectivement.

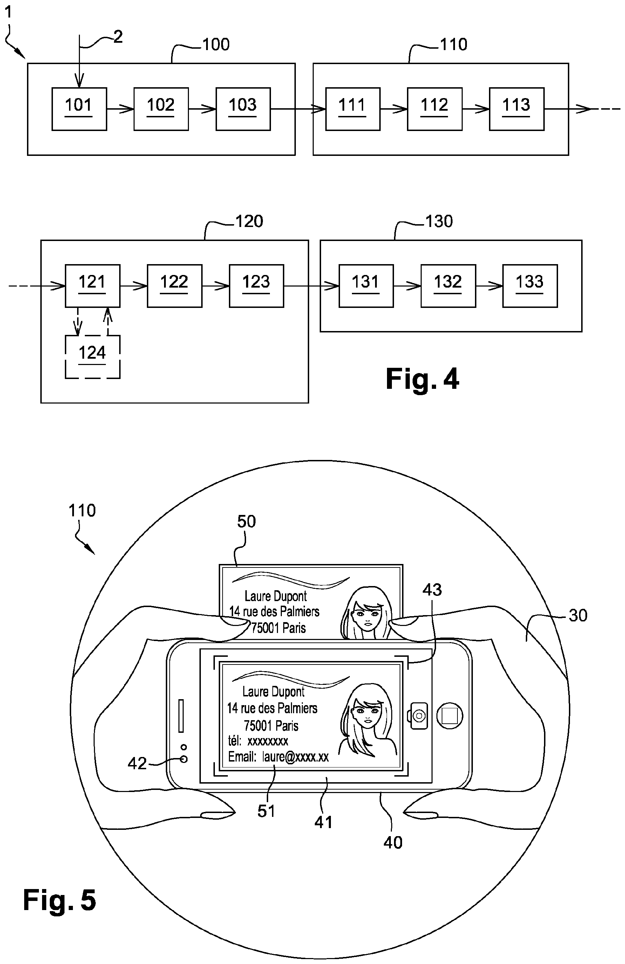

Sur les figures 4 à 12 est représentée la méthode de détermination 1 conforme à l'invention. Cette méthode 1 permet de déterminer une taille particulière de téterelle 10 convenant à une utilisatrice 30 particulière, parmi différentes tailles disponibles, telles qu'illustrées par les disques 21, 22, 23 et 24.

La méthode 1 comprend plusieurs étapes successives 100, 110, 120 et 130, comprenant elles-mêmes les sous-étapes 101, 102, 103, 111, 112, 113, 121, 122, 123, 131, 132 et 133 détaillées ci-après.

Avantageusement, la méthode 1 peut être mise en oeuvre à l'aide d'une application 2 dédiée, exécutée sur un terminal mobile personnel 40 appartenant à l'utilisatrice 30. Le terminal 40 comprend un écran 41 et un dispositif de capture d'image 42. Sur l'exemple des figures 5 à 9, le terminal 40 est un téléphone numérique (« smartphone » en anglais) et le dispositif 42 comprend deux objectifs disposés des deux côtés du téléphone.

L'étape préliminaire 100 consiste à activer l'application 2 et comprend les sous-étapes 101, 102 et 103.

La sous-étape 101 consiste à charger l'application 2 sur le terminal de l'utilisatrice. De préférence, l'application 2 est téléchargée depuis un site internet, notamment une bibliothèque d'applications pour terminaux mobiles.

La sous-étape 102 consiste à installer l'application 2 sur le terminal de l'utilisatrice. Généralement, l'installation est réalisée automatiquement à la fin du téléchargement.

La sous-étape 103 consiste à exécuter l'application 2 sur le terminal de l'utilisatrice. Généralement, le lancement est effectué manuellement par l'utilisatrice, lorsque celle-ci souhaite déterminer la taille de téterelle 10 qui lui convient. Idéalement, l'application 2 devrait être utilisée après une tétée ou l'expression du lait. Cependant, l'application 2 est susceptible d'être utilisée lorsque le mamelon est au repos, alors que l'utilisatrice 30 ne dispose pas encore d'une téterelle 10. Dans ce cas, il convient d'ajouter 2 millimètres aux mesures effectuées.

A la fin de l'étape 100, l'application 2 est active. L'utilisatrice 30 est invitée à consulter ou non le manuel d'utilisation de l'application 2. Après consultation du manuel, ou si elle ne souhaite pas consulter le manuel, l'utilisatrice 30 est invitée à cibler un repère 50 puis appuyer sur le bouton caméra, ce qui correspond à l'étape 110.

Sur la figure 5 est représentée l'étape 110, consistant à caractériser un repère 50 de dimensions déterminées, affiché sous forme d'un repère numérique 51 sur l'écran 41 du terminal 40. L'étape 110 comprend les sous-étapes 111, 112 et 113.

En pratique, le repère 50 non-virtuel est choisi avec des dimensions connues. De préférence, le repère 50 présente une forme rectangulaire, avec une largeur et une hauteur données. Encore de préférence, le repère 50 présente les dimensions standards d'une carte à puce au format ID-1, à savoir une largeur de 85,6 millimètres et une hauteur de 54 millimètres. A titre d'exemple, le repère 50 peut être une carte de crédit bancaire, une carte de sécurité sociale (carte « Vitale ») ou une carte de fidélité d'un magasin, présentant les dimensions standards d'une carte à puce ID-1.

La sous-étape 111 consiste à positionner le repère 50 devant un objectif du dispositif 42. Le repère 50 est alors automatiquement affiché sous forme de repère numérique 51 sur l'écran 41.

La sous-étape 112 consiste à positionner le repère numérique 51 dans un cadre 43 affiché sur l'écran 41. Le cadre 43 est fixe et présente des dimensions déterminées. Le cadre 43 peut être matérialisé par les quatre coins d'un rectangle, ou par un rectangle complet, affiché sur l'écran 41.

La sous-étape 113 consiste à actionner le dispositif 42, lorsque le repère numérique 51 est positionné dans le cadre 43, pour prendre une photographie du repère 50. Cette photographie doit être de bonne qualité, c'est-à-dire présenter une bonne netteté et une bonne luminosité, sans reflets.

A la fin de l'étape 110, l'application 2 mémorise les caractéristiques du repère 50, en l'espèce sa forme et ses dimensions. L'application 2 est capable d'établir une correspondance entre les dimensions du repère 50 tenu en main par l'utilisatrice 30 et les dimensions du repère numérique 51 affiché sur l'écran 41.

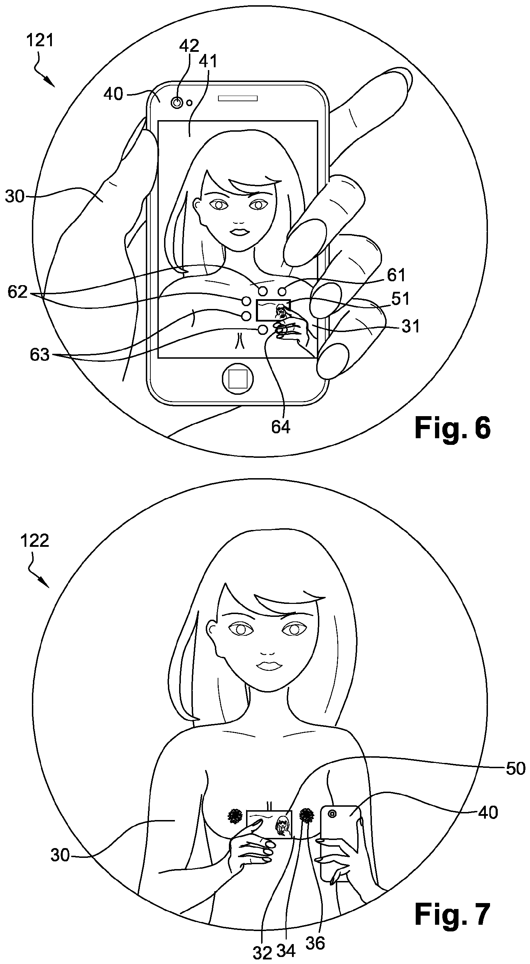

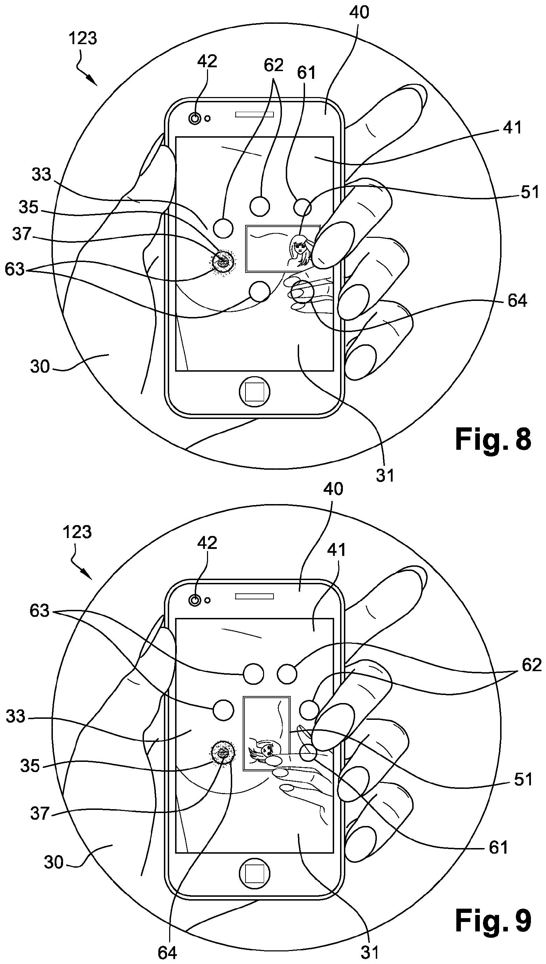

Sur les figures 6 à 10 est représentée l'étape 120, comprenant les sous-étapes 121, 122 et 123. Comme montré en particulier aux figures 7 et 8, cette étape 120 consiste à comparer les dimensions d'une représentation de mamelon 37 de l'utilisatrice 30 sur l'écran 41, avec des cercles colorés 61, 62, 63 et 64 de différents diamètres affichés à côté du repère numérique 51 sur l'écran 41 du terminal 40, alors que le repère 50 est positionné à côté du mamelon 36 de l'utilisatrice 30. L'étape 120 peut également comprendre une sous-étape d'aide 124, optionnelle.

La sous-étape 121 illustrée à la figure 6 consiste à générer automatiquement des cercles 61, 62, 63 et 64 de différents diamètres à côté du repère numérique 51 sur l'écran 41, en réalité augmentée. Si les cercles 61, 62, 63 et 64 n'apparaissent pas sur l'écran 41, il convient de répéter l'étape 110, éventuellement en changeant de repère 50. A ce stade, l'utilisatrice 30 a la possibilité d'afficher une page d'aide sur l'application 2, ce qui correspond à la sous-étape 124. La page d'aide explique les différentes raisons pour lesquelles les cercles peuvent ne pas apparaître, et les mesures possibles pour y remédier.

De même que les dimensions du repère virtuel 51 sont proportionnelles à celles du repère 50, les différents diamètres des cercles colorés 61, 62, 63 et 64 sont proportionnels aux différents diamètres des disques colorés 21, 22, 23 et 24, correspondant aux différentes tailles de téterelles 11, 12, 13 et 14. Lorsque l'éloignement entre le dispositif 42 et le repère 50 change, alors les dimensions du repère numérique 51 et des cercles 61, 62, 63 et 64 changent sur l'écran 41. Par commodité, l'écran 41 peut afficher deux cercles 62 et deux cercles 63.

A ce stade, l'utilisatrice 30 doit se tenir sein nu face à son terminal 40. Plus précisément, l'utilisatrice 30 présente au dispositif 42 au moins un sein 32 nu comprenant une aréole 34 et un mamelon 36. Des représentations 31, 33, 35 et 37 de l'utilisatrice 30, de son sein 32, de son aréole 34 et de son mamelon 36, respectivement, sont affichées sur l'écran 41 avec des dimensions proportionnelles à la réalité.

La sous-étape 122 illustrée à la figure 7 consiste à positionner le repère 50 à côté du mamelon 36 de l'utilisatrice 30. Le mamelon 36 et le repère 50 se situent alors à la même distance du dispositif de capture d'image 42. Sur l'écran 41, le repère numérique 51 et les cercles 61, 62, 63 et 64 sont ainsi positionnés à côté de la représentation de mamelon 37.

La sous-étape 123 illustrée aux figures 8 et 9 consiste à déplacer le repère 50 autour du mamelon 36, ce qui déplace le repère numérique 51 et les cercles 61, 62, 63 et 64 autour de la représentation de mamelon 37 sur l'écran 41. L'utilisatrice 30 compare alors les diamètres des cercles 61, 62, 63 et 64 avec les dimensions de la représentation de mamelon 37. Plus précisément, l'utilisatrice 30 superpose tour à tour les cercles 61, 62, 63 et 64 avec la représentation de mamelon 37 sur l'écran 41, jusqu'à identifier le cercle 61, 62, 63 ou 64 dont le diamètre s'accorde avec les dimensions de la représentation de mamelon 37.

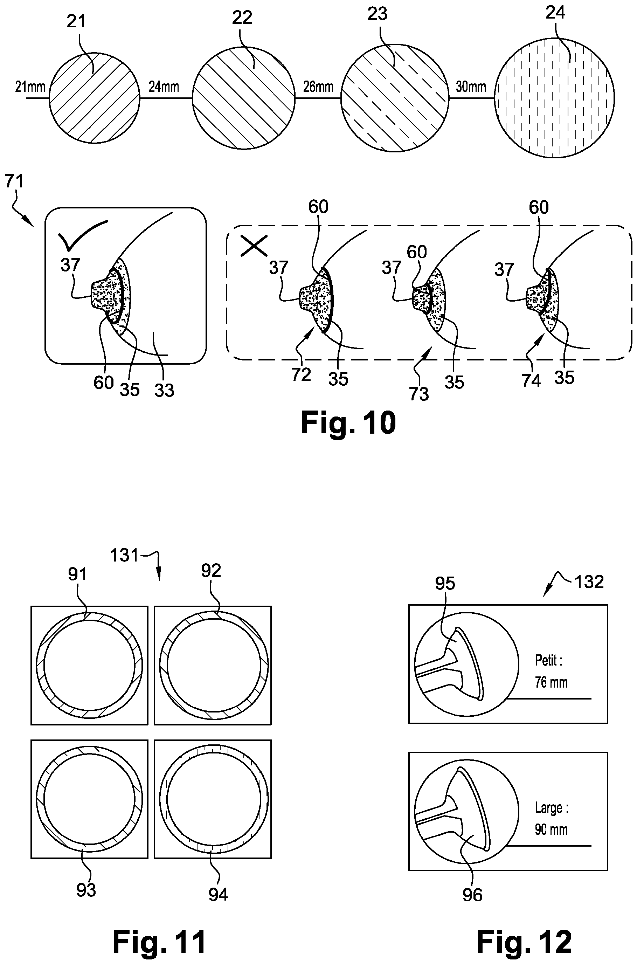

La figure 10 montre une vignette du manuel d'utilisation conçue pour aider l'utilisatrice 30, lorsque son mamelon 36 est au repos, à déterminer quel cercle 61, 62, 63 ou 64 s'accorde au mieux avec les dimensions de la représentation de mamelon 37 sur l'écran 41. Pour tenir compte du fait que le mamelon 36 de l'utilisatrice 30 est au repos, il convient en effet d'ajouter 2 millimètres aux mesures effectuées. La vignette montre les différents disques 21, 22, 23 et 24 pour rappel. La vignette montre également un positionnement correct 71 d'un cercle 60 autour de la représentation de mamelon 37, entre la périphérie de l'aréole 35 et du mamelon 37. La page d'aide montre également des positionnements incorrects 72, 73 et 74 du cercle 60 sur l'aréole 35. Le positionnement 72 correspond à un cercle 60 disposé en périphérie de l'aréole 35. Le positionnement 73 correspond à un cercle 60 disposé contre la base de la représentation de mamelon 37. Le positionnement 74 correspond à un cercle 60 disposé de travers sur l'aréole 35.

Sur les figures 11 et 12 est représentée l'étape 130, consistant à sélectionner la taille 21, 22, 23 ou 24 de téterelle 10 convenant à l'utilisatrice 30, en fonction du cercle 61, 62, 63 ou 64 correspondant retenu par l'utilisatrice 30 à la fin de l'étape 120. L'étape 130 comprend les sous-étapes 131, 132 et 133.

La sous-étape 131 illustrée à la figure 11 consiste à sélectionner un cercle coloré 91, 92, 93 ou 94 en appuyant sur l'écran 41, en fonction du cercle coloré 61, 62, 63 ou 64 correspondant qui a été retenu par l'utilisatrice 30 à la fin de l'étape 120.

La sous-étape 132 illustrée à la figure 12 consiste à sélectionner un gabarit 95 ou 96 de téterelle 10 en appuyant sur l'écran 41, en fonction de la forme du sein 32 de l'utilisatrice 30. Si l'utilisatrice 30 a un sein 32 pointu en forme de poire, l'application 2 lui propose de choisir le petit gabarit 95, tandis que si l'utilisatrice 30 a un sein 32 arrondi en forme de pomme, l'application 2 lui propose de choisir le gabarit large 96.

La sous-étape 133, non représentée sur les figures, consiste à indiquer à l'utilisatrice 30 la taille 21, 22, 23 ou 24 et le gabarit 95 ou 96 de téterelle 10 qui lui conviennent le mieux. Cette indication peut se présenter sous forme de mémo récapitulatif, proposant un ou plusieurs produits disponibles dans le commerce. Par exemple, l'application 2 peut afficher les données suivantes sur l'écran 41 :
- 21 mm Large
- Vous avez choisi le diamètre intérieur 21 mm (violet) pour votre téterelle avec un embout confort Large.
- Rendez-vous en pharmacie pour obtenir le KIT EXPRESSION KOLOR ® de la gamme Kittet qui vous convient, disponible en simple ou double pompage.

L'utilisatrice 30 peut alors se rendre en pharmacie pour obtenir le produit désiré.

En pratique, la méthode 1 peut mettre en oeuvre des sous-étapes différentes de celles décrites ci-dessus sans sortir du cadre de l'invention. Ainsi, la méthode 1 peut être adaptée en termes d'ergonomie, de fonctionnalités et de performance.

## Revendications

1. Méthode de détermination (1) d'une taille (21, 22, 23, 24) de téterelle (10), par une utilisatrice (30) munie d'un terminal mobile personnel (40) équipé d'un écran (41) et d'un dispositif de capture d'image (42), la méthode comprenant les étapes successives suivantes :
- une étape (110) consistant à caractériser un repère (50) de dimensions déterminées, affiché sous forme d'un repère numérique (51) sur l'écran (41) du terminal (40) ;
- une étape (120) consistant à comparer les dimensions d'une représentation de mamelon (37) de l'utilisatrice (30) avec des cercles (61, 62, 63, 64) de différents diamètres affichés à côté du repère numérique (51) sur l'écran (41) du terminal (40), alors que le repère (50) est positionné à côté d'un mamelon (36) de l'utilisatrice (30) ; et
- une étape (130) consistant à sélectionner la taille (21, 22, 23, 24) de téterelle (10) convenant à l'utilisatrice (30), en fonction du cercle (61, 62, 63, 64) retenu à la fin de l'étape (120) précédente.

2. Méthode de détermination (1) selon la revendication précédente, **caractérisée en ce que** l'étape (110) consistant à caractériser le repère (50) de dimensions déterminées comprend les sous-étapes successives suivantes :
- une sous-étape (111) consistant à positionner le repère (50) face au dispositif de capture d'image (42), de sorte que le repère (50) est automatiquement affiché sous forme de repère numérique (51) sur l'écran (41) ;
- une sous-étape (112) consistant à positionner le repère numérique (51) dans un cadre (43) affiché sur l'écran (41) ; et
- une sous-étape (113) consistant à actionner le dispositif de capture d'image (42), lorsque le repère numérique (51) est positionné dans le cadre (43).

3. Méthode de détermination (1) selon l'une des revendications précédentes, **caractérisée en ce que** l'étape (120) consistant à comparer les dimensions de la représentation de mamelon (37) avec les cercles (61, 62, 63, 64) de différents diamètres comprend les sous-étapes successives suivantes :
- une sous-étape (121) consistant à générer les cercles (61, 62, 63, 64) de différents diamètres à côté du repère numérique (51) sur l'écran (41) ;
- une sous-étape (122) consistant à positionner le repère (50) à côté du mamelon (36) de l'utilisatrice (30), de sorte que le repère numérique (51) et les cercles (61, 62, 63 et 64) sont positionnés à côté de la représentation de mamelon (37) sur l'écran (41) ; et
- une sous-étape (123) consistant à déplacer le repère (50) autour du mamelon (36) de l'utilisatrice (30), jusqu'à identifier le cercle (61, 62, 63, 64) dont le diamètre s'accorde avec les dimensions de la représentation de mamelon (37) sur l'écran (41).

4. Méthode de détermination (1) selon l'une des revendications précédentes, **caractérisée en ce que** l'étape (120) consistant à comparer les dimensions de la représentation de mamelon (37) avec les cercles (61, 62, 63, 64) de différents diamètres comprend une sous-étape (124) consistant à afficher une page d'aide, au choix de l'utilisatrice (30).

5. Méthode de détermination (1) selon l'une des revendications précédentes, **caractérisée en ce que** l'étape (130) consistant à sélectionner la taille (21, 22, 23, 24) de téterelle (10) convenant à l'utilisatrice (30) comprend les sous-étapes successives suivantes :
- une sous-étape (131) consistant à sélectionner un cercle coloré (91, 92, 93, 94) en appuyant sur l'écran (41), en fonction du cercle (61, 62, 63, 64) retenu par l'utilisatrice (30) ; et
- une sous-étape (133) consistant à indiquer à l'utilisatrice (30) la taille (21, 22, 23 ou 24) de téterelle (10) qui lui convient le mieux.

6. Méthode de détermination (1) selon la revendication 5, **caractérisée en ce que** l'étape (130) consistant à sélectionner la taille (21, 22, 23, 24) de téterelle (10) convenant à l'utilisatrice (30) comprend :
- une sous-étape (132) additionnelle consistant à sélectionner un gabarit (95, 96) de téterelle (10) en appuyant sur l'écran (41), en fonction de la forme du sein de l'utilisatrice (30) ; et
- la sous-étape (133) consistant à indiquer à l'utilisatrice (30) la taille (21, 22, 23 ou 24) et également le gabarit (95, 96) de téterelle (10) qui lui conviennent le mieux.

7. Méthode de détermination (1) selon l'une des revendications précédentes, **caractérisée en ce que** le repère (50) présente les dimensions standards d'une carte à puce ID-1.

8. Produit programme informatique (2), comprenant des portions de code de programme enregistrées sur le terminal (40) de l'utilisatrice (30), pour la mise en oeuvre des étapes de la méthode de détermination (1) exécutables par le terminal (40) de l'utilisatrice (30) selon l'une des revendications précédentes, lorsque ledit programme est exécuté sur le terminal (40) de l'utilisatrice (30).

## Patentansprüche

1. Methode zur Bestimmung (1) der Größe (21, 22, 23, 24) einer Brustkappe (10), durch eine Benutzerin (30), die ein mobiles Endgerät (40) mit einem Bildschirm (41) und einer Bilderfassungsvorrichtung (42) besitzt, wobei die Methode aus den nachstehenden, aufeinanderfolgenden Schritten besteht:
- Schritt (110) besteht darin, eine Markierung (50) mit festgelegten Maßen zu bestimmen, die in Form einer numerischen Markierung (51) auf dem Bildschirm (41) des Endgerätes (40) angezeigt wird;
- Schritt (120) besteht darin, die Maße einer Darstellung der Brustwarze (37) der Benutzerin (30) mit Kreisen (61, 63, 64) unterschiedlicher Durchmesser zu vergleichen, die neben der numerischen Markierung (51) am Bildschirm (41) des Endgerätes (40) angezeigt werden, während die Markierung (50) neben einer Brustwarze (36) der Benutzerin (30) angezeigt wird; und
- Schritt (130) besteht darin, die Größe (21, 22, 23, 24) der Brustkappe (10), die für die Benutzerin (30) passt, entsprechend dem Kreis (61, 62, 63, 64), auszuwählen, der am Ende des vorangehenden Schritts (120) ausgewählt wurde.

2. Bestimmungsmethode (1) nach dem vorangehenden Anspruch, **dadurch gekennzeichnet, dass** der Schritt (110), der in der Bestimmung der Markierung (50) mit festgelegten Maßen besteht, die nachstehenden, aufeinanderfolgenden Unterschritte umfasst:
- Unterschritt (111) besteht darin, die Markierung (50) gegenüber der Bilderfassungsvorrichtung (42) zu positionieren, so dass die Markierung (50) automatisch in Form einer numerischen Markierung (51) am Bildschirm (41) angezeigt wird;
- Unterschritt (112) besteht darin, die numerische Markierung (51) in einem am Bildschirm (41) angezeigten Rahmen (43) zu positionieren; und
- Unterschritt (113) besteht darin, die Bilderfassungsvorrichtung (42) zu betätigen, wenn die numerische Markierung (51) im Rahmen (43) positioniert ist.

3. Bestimmungsmethode (1) nach dem vorangehenden Anspruch, **dadurch gekennzeichnet, dass** der Schritt (120), der im Vergleich der Maße der Darstellung der Brustwarze (37) mit Kreisen (61, 62, 63, 64) unterschiedlichen Durchmessers besteht, die nachstehenden aufeinanderfolgenden Unterschritte umfasst:
- Unterschritt (121) besteht darin, Kreise (61, 62, 63, 64) unterschiedlichen Durchmessers, neben einer numerischen Markierung (51) auf dem Bildschirm (41) zu erzeugen;
- Unterschritt (122) besteht darin, die Markierung (50) neben der Brustwarze (36) der Benutzerin (30) zu positionieren, so dass die numerische Markierung und die Kreise (61, 62, 63, 64) sich neben der Darstellung der Brustwarze (37) auf dem Bildschirm (41) befinden; und
- Unterschritt (123) besteht darin, die Markierung (50) um die Brustwarze (36) der Benutzerin (30) zu verschieben, bis der Kreis (61, 62, 63, 64) erkannt ist, dessen Durchmesser zu den Maßen der Darstellung der Brustwarze (37) am Bildschirm (41) passt.

4. Bestimmungsmethode (1) nach einem der vorhergehenden Ansprüche, **dadurch gekennzeichnet, dass** der der Schritt (120), der im Vergleich der Maße der Darstellung der Brustwarze (37) mit Kreisen (61, 62, 63, 64) unterschiedlichen Durchmessers besteht, einen Unterschritt (124) enthält, der darin besteht, eine Hilfsseite, nach Wahl der Benutzerin (30) anzuzeigen.

5. Bestimmungsmethode (1) nach einem der vorhergehenden Ansprüche, **dadurch gekennzeichnet, dass** der Schritt (130), der darin besteht, die Größe (21, 22, 23, 24) der Brustkappe (10) auszuwählen, die für die Benutzerin (30) passt, die nachstehenden aufeinanderfolgenden Unterschritte umfasst:
- Unterschritt (131) besteht darin, einen farbigen Kreis (91, 92, 93, 94) auszuwählen, durch Antippen des Bildschirms (41) entsprechend dem von der Benutzerin (30) ausgewählten Kreis (61, 62, 63, 64); und
- Unterschritt (133) besteht darin, der Benutzerin (30) die Größe (21, 22, 23 oder 24) der Brustkappe (10) mitzuteilen, die ihr am besten passt.

6. Bestimmungsmethode (1) nach Anspruch 5, **dadurch gekennzeichnet, dass** der Schritt (130), der darin besteht, die Größe (21, 22, 23, 24) der Brustkappe (10) der Benutzerin (30) auszuwählen, umfasst:
- einen zusätzlichen Unterschritt (132) der darin besteht, eine Schablone (95, 96) für die Brustkappe (10) auszuwählen, durch Antippen des Bildschirms (41) entsprechend der Form der Brust der Benutzerin (30); und
- einen Unterschritt (133), der darin besteht der Benutzerin (30) die Größe (21, 22, 23, oder 24) sowie die Schablone (95, 96) der Brustkappe (10) mitzuteilen, die ihr am besten passt.

7. Bestimmungsmethode (1) nach einem der vorhergehenden Ansprüche, **dadurch gekennzeichnet, dass** die Markierung (50) die Standardmaße einer ID-1-Chipkarte aufweist.

8. Softwareprodukt (2), das die Abschnitte des Programmcodes erhält, die auf dem Endgerät (40) der Benutzerin (30) gespeichert sind, zur Umsetzung der einzelnen Schritt der Bestimmungsmethode (1) die vom Endgerät (40) der Benutzerin (30) auszuführen sind, nach einem der vorhergehenden Ansprüchen, wenn dieses Programm auf dem Endgerät (40) der Benutzerin (30) ausgeführt wird.

## Claims

1. A determination method (1) of a size (21, 22, 23, 24) of a breastshield (10) by a user (30) provided with a personal mobile terminal (40) equipped with a screen (41) and an image capture device (42), the method comprising the following successive steps:
- a step (110) which consists in the characterizing a reference (50) having determined dimensions, displayed in the form of a digital reference (51) on the screen (41) of the terminal (40);
- a step (120) consisting in comparing the dimensions of a representation of a nipple (37) of the user (30) with circles (61, 62, 63, 64) having different diameters displayed alongside a digital reference (51) on the screen (41) of the terminal (40), whilst the reference (50) is placed alongside a nipple (36) of the user (30); and
- a step (130) consisting in selecting the size (21, 22, 23, 24) of the breastshield (10) suiting the user (30), depending on the circle (61, 62, 63, 64) chosen at the end of the previous step (120).

2. A determination method (1) according to the previous claim, **characterized in that** the step (110) consisting in characterizing the reference (50) having determined dimensions comprises the following successive substeps:
- a substep (111) consisting in positioning the reference (50) opposite the image capture device (42) in such a way that the reference (50) is displayed automatically in the form of a digital reference (51) on the screen (41);
- a substep (112) consisting in positioning the digital reference (51) in a box (43) displayed on the screen (41); and
- a substep (113) consisting in actuating the image capture device (42) when the digital reference (51) is placed inside the frame (43).

3. A determination method (1) according to one of the previous claims, **characterized in that** the step (120) consisting in comparing the representation dimensions of the nipple (37) with the circles (61, 62, 63, 64) having different diameters includes the following successive substeps:
- a substep (121) which consists in generating circles (61, 62, 63, 64) having different diameters alongside the digital reference (51) on the screen (41);
- a substep (122) which consists in positioning the reference (50) alongside the nipple (36) of the user (30), so that the digital reference (51) and the circles (61, 62, 63 and 64) are positioned alongside the representation of the nipple (37) on the screen (41); and
- a substep (123) which consists in moving the reference (50) around the nipple (36) of the user (30), until identification of the circle (61, 62, 63, 64) with a diameter matching the dimensions of the representation of the nipple (37) on the screen (41) occurs.

4. A method of determination (1) according to one of the previous claims, **characterized in that** the step (120) consisting in comparing the dimensions of the representation of the nipple (37) with the circles (61, 62, 63, 64) of different diameters includes a substep (124) consisting in displaying a help page as chosen by the user (30).

5. A method of determination (1) according to one of the previous claims, **characterized in that** the step (130) consists in selecting the size (21, 22, 23, 24) of the breastshield (10) suiting the user (30) comprises one of the following successive substeps:
- a substep (131) consisting in selecting a colored circle (91, 92, 93, 94) by pressing on the screen (41), depending on the circle (61, 62, 63, 64) chosen by the user (30); and
- a substep (133) consisting in indicating to the user (30) the size (21, 22, 23 or 24) of the breastshield (10) that suits her best.

6. A method of determination (1) according to the claim 5, **characterized in that** the step (130) consisting in selecting the size (21, 22, 23, 24) of the breastshield (10) suiting the user (30) includes:
- an additional substep (132) which consists in selecting a gauge (95, 96) for the breastshield (10) by pressing on the screen (41). according to the shape of the breast of the user (30); and
- a substep (133) consisting in indicating to the user (30) the size (21, 22, 23 or 24) and also the gauge (95, 96) of the breastshield (10) that suits her best.

7. A method of determination (1) according to one of the previous claims, **characterized in that** the reference (50) has the standard dimensions of a smartcard ID-1.

8. A computer program product (2) comprising portions of program code recorded on the terminal (40) of the user (30) for the implementing of the steps of the determination method (1) which can be executed by the terminal (40) of the user (30) according to one of the previous claims, when the said program is executed on the terminal (40) of the user (30).
